# EUROPEAN PATENT APPLICATION

(11) **EP 2 508 222 A1**
(43) Date of publication of application: **10.10.2012**
(21) Application number: 12305410.8
(22) Date of filing: 06.04.2012
(51) Int. Cl.: A61M 25/10

(54) **Balloon catheter for launching drug delivery device**

(30) Priority: 08.04.2011 US 201161473507 P; 30.03.2012 US 201213436423
(71) Applicant: Sanovas, Inc., Sausalito, CA 94965 (US)
(72) Inventor: Gerrans, Lawrence J., SAN ANSELMO, California 94960 (US); Gunday, Erhan H., GREAT NECK, New York 11021 (US)
(74) Representative: Denjean, Eric

(57) **Abstract**

An apparatus for delivering a therapeutic and/or diagnostic agent to tissue within a bodily cavity is provided herein. The apparatus may include a catheter (22) assembly including a catheter (22) and a delivery balloon (36) having an expandable material (38) disposed thereon, wherein the expandable material may be an absorbent (40) at least partially charged with a therapeutic and/or diagnostic agent.

## Description

### FIELD OF THE INVENTION

The present invention relates to methods and systems for delivering therapeutic and/or diagnostic agents to specific cellular locations within and adjacent to bodily tissues and cavities. More specifically, the invention relates to a method and system of delivery of diagnostic and/or therapeutic agents to bodily tissues and cavities via a balloon catheter having an expandable absorbent or stent charged with the therapeutic and/or diagnostic agent, disposed thereon.

### BACKGROUND OF THE INVENTION

In diagnosing and treating diseases of various body cavities and organs, it is necessary to deliver diagnostic and/or therapeutic agents to the organs at specified locations. The most common routes of drug delivery include non-invasive peroral (through the mouth), topical (skin), transmucosal (nasal, buccal/sublingual, vaginal, ocular and rectal) and inhalation. However, many therapeutic and diagnostic agents in general may not be delivered using these routes because the agents may be susceptible to enzymatic degradation or cannot be absorbed into the systemic circulation efficiently due to molecular size and charge issues, and thus, will not be therapeutically effective. For this reason, many such drugs have to be delivered by injection.

There are several known problems associated with the injection process. One of such problems is undesirable extravasation of the diagnostic or therapeutic agents into tissue, which is particularly prevalent with intravenously injected agents. Extravasation generally refers to leakage of fluids out of a container, and more specifically refers to leakage of intravenous drugs from a vein into surrounding tissues, resulting in injury to the tissues. Once the intravenous extravasation has occurred, damage can continue for months and involve nerves, tendons and joints. If treatment is delayed, surgical debridement, skin grafting, and even amputation have been known to be the unfortunate consequences.

Occurrence of extravasation is possible with all intravenous drugs, but it is a particularly significant problem with cytoxic drugs used for treatment of cancer (i.e. during chemotherapy).

Chemotherapy is the general term for any treatment involving the use of chemical agents to stop cancer cells from growing. Chemotherapy can eliminate cancer cells at sites great distances from the original cancer. As a result, chemotherapy is considered a systemic treatment. More than half of all people diagnosed with cancer receive chemotherapy. A chemotherapy regimen (a treatment plan and schedule) usually includes drugs to fight cancer plus drugs to help support completion of the cancer treatment.

Chemotherapy can be administered through a vein, injected into a body cavity, or delivered orally in the form of a pill, depending on which drug is used. Chemotherapy works by destroying cancer cells. Unfortunately, it cannot tell the difference between a cancer cell and some healthy cells. Thus, chemotherapy often eliminates not only the fast-growing cancer cells, but also other fast-growing cells in the body, including hair and blood cells. Some cancer cells grow slowly while others grow rapidly. As a result, different types of chemotherapy drugs target the growth patterns of specific types of cancer cells.

Each chemotherapy drug works differently and is effective at a specific time in a life cycle of the cell it targets. Brachytherapy, sometimes called seed implantation, is an outpatient procedure used in the treatment of different kinds of cancer. The radioactive "seeds" are carefully placed inside of the cancerous tissue and positioned in a manner that will attack the cancer most efficiently. The radioactive seeds are about the size of a grain of rice, and give off radiation that travels only a few millimeters to kill nearby cancer cells. There are two different kinds of brachytherapy: permanent, when the seeds remain inside the body, and temporary, when the seeds are inside of the body and are then removed. With permanent implants (e.g. prostate), the radioactivity of the seeds typically decays with time.

The other type of chemotherapy is when cytotoxic agents are delivered intravenously. Veins of people receiving chemotherapy are often fragile, mobile, and difficult to cannulate. Patients who receive chemotherapy at the same site as radiotherapy may experience a reactivation of skin toxicity known as a "recall" phenomenon. Patients who have had previous radiation therapy at the site of injection may develop severe local reactions from extravasated cytotoxic drugs. Cytotoxic drugs also have the potential to cause cutaneous abnormalities in areas that have been damaged previously by radiation, even in areas that are distant from the injection site. Patients who have had an extravasation and receive further chemotherapy in a different site may experience an exacerbation of tissue damage in the original site.

Furthermore, areas of previous surgery where the underlying tissue is likely to be fibrosed and toughened dramatically present an increased risk of extravasation. Radical mastectomy, axillary surgery or lymph node dissection may impair circulation in a particular limb. This reduces venous flow and may allow intravenous solutions to pool and leak around the site of cannulation.

Some chemotherapy drugs often never reach the tumors they are intended to treat because the blood vessels feeding the tumors are abnormal. A tumor's capillaries (small blood vessels that directly deliver oxygen and nutrients to cancer cells) can be irregularly shaped, being excessively thin in some areas and forming thick, snarly clumps in others. These malformations create a turbulent, uneven blood flow, so that too much blood goes to one region of the tumor, and too little to another. In addition, the capillary endothelial cells lining the inner surface of tumor capillaries, normally a smooth, tightly-packed sheet, have gaps between them, causing vessel leakiness.

The systemic and intravenous side effects of chemotherapy coupled with the limited effect of systemic administration due to abnormal characteristics of tumor blood vessels have given the scientific community pause, in searching for more direct, localized and biologic solutions. Accordingly, the oncology literature has become increasingly populated with articles espousing prospective benefits and positive outcomes of intra-tumoral chemotherapy. A direct administration of cytotoxic drugs such as Mytomycin, Mytomycin-C, Bleomycin, Fluorouracil, Mitoxantrone, Cisplatin, and Avastin in endobronchial intra-tumoral chemotherapy has been done experimentally via direct injection of the agent into the endobronchial tumor. In these cases, the tumor was reported to have died and been subsequently removed.

However, while some experimental uses of the localized delivery of cytotoxic drugs have been attempted, there has been little implementation of such drug delivery in practice, possibly due to numerous problems associated with such delivery. First, it is often necessary to deliver cytotoxic drugs to remote and not easily accessible blood vessels and other lumens within body organs, such as lungs. It is also important to be able to deliver defined doses of the cytotoxic substances because such substances are often very expensive or are capable of causing serious harm if delivered in excess. Moreover, the existing methods lack the ability to contain the cytotoxic agent and/or radiation therapy and mitigate collateral damage to non-affected anatomy and structures.

Several devices have been proposed for a targeted delivery of drugs to internal bodily cavities. For example, U.S. Patent No. 5,397,307 to Goodwin and U.S. Patent Application No. 2008/0208118 by Goldman propose using a catheter with two balloons to deliver the therapeutic agent. In these systems, first and second balloons are inflated to create a chamber between them, and a therapeutic agent is then introduced into this chamber. While useful for confining the delivery of the agent to a specific target site, these systems are not particularly efficient at infusing the relevant biological material with the drug. Instead, the catheter may need to remain in place for an unnecessarily long period of time while the infusion of the drug into the biological material is allowed to take place. This is undesirable, especially in applications such as pulmonology, where the patient's respiratory passage has been somewhat restricted by the device. Further, this can result in some of the agent never being infused into the targeted material and instead remaining in the cavity and, after the balloon catheter is removed, subsequently migrating to other undesired portions of the body.

Another solution that has been proposed is using a catheter with a singular balloon coated with a sponge-like polymer material that is saturated with a diagnostic or therapeutic agent, such as those disclosed in U.S. Patent Publication No. 2003/00114791 to Rosethal et al., U.S. Patent No. 7,462,165 to Ding et al., U.S. Patent No. 6,129,705 to Grantz, U.S. Patent No. 5,304,121 to Sahatjian and U.S. Patent No. 5,674,192 to Sahatjian et al. In these systems, a delivery balloon coated with a sponge-like polymer material that is saturated with a diagnostic or therapeutic agent is inserted into a body lumen and expanded against the wall of the body lumen. After some period of time, the delivery balloon is deflated and the balloon with the sponge-like material is removed from the body. This system is undesirable because the delivery balloon must remain in place for an unnecessarily long period of time while the infusion of the drug from the sponge-like coating into the biological material is allowed to take place. Ding et al., Grantz, Sahatjian and Sahatjian et al. further teach the use of a catheter with a single balloon having a stent saturated with a diagnostic or therapeutic agent, disposed thereon.

What is desired, therefore, is a system for delivering therapeutic and/or diagnostic agents to bodily tissues, tumors, and other biological materials that can locally deliver the agent to a specific target site. What is further desired is a system for delivering therapeutic and/or diagnostic agents to bodily tissues, tumors, and other biological materials over an extended period of time, without requiring prolonged occlusion of air and blood flow through the bodily passage.

### SUMMARY OF THE INVENTION

Accordingly, it is an object of the present invention to provide a balloon catheter system that can deliver therapeutic and/or diagnostic agents to bodily tissues, tumors, and other biological materials from within bodily cavities, for prolonged periods of time.

It is another object of the present invention to provide a nested balloon catheter system that can locally deliver therapeutic and/or diagnostic agents to bodily tissues, tumors, and other biological materials from within bodily cavities, and prevent extravasation of agents to unintended tissues.

It is a further object of the present invention to provide a balloon catheter system that can target specific areas for the delivery of therapeutic and/or diagnostic agents to bodily tissues, tumors, and other biological materials.

It is another object of the present invention to provide a balloon catheter system for delivering therapeutic and/or diagnostic agents to bodily tissues, tumors, and other biological materials that permits the passage of bodily fluids through the system.

It is yet another object of the present invention to provide a balloon catheter system for delivering therapeutic and/or diagnostic agents to bodily tissues, tumors, and other biological material that provides visualization within the bodily cavity.

In order to overcome the deficiencies of the prior art and to achieve at least some of the objects and advantages listed, the invention may comprise a method of delivery of a therapeutic and/or diagnostic agent to tissue, comprising the steps of: inserting a catheter assembly into a bodily cavity, the catheter assembly comprising a catheter and a delivery balloon having an expandable material disposed thereon, the expandable material comprising an absorbent at least partially charged with a therapeutic and/or diagnostic agent; and delivering said therapeutic and/or diagnostic agent to tissue by inflating the delivery balloon by supplying fluid thereto until the expandable material adopts an expanded state in which the expandable material contacts tissue and is implanted in the bodily cavity. After at least some of said agent has been absorbed by the tissue, at least a portion of the implanted expandable material may be recharged with a therapeutic and/or diagnostic agent.

The method may further include the steps of deflating the delivery balloon and removing the delivery balloon from the bodily cavity while the expandable material remains in the expanded state and implanted in the bodily cavity. After at least some of said agent has been absorbed by the tissue, at least a portion of the implanted expandable material may be recharged with a therapeutic and/or diagnostic agent. The expandable material may further include a micro RF tag.

The method may further include the step of curing the expandable material to adopt a rigid shape within the bodily cavity. This curing step my include introducing a probe into the catheter to apply a curing treatment to the expandable material.

The absorbent may also be retained on the delivery balloon in a contracted state with at least one string. The step of inflating the delivery balloon may cause the at least one string to break such that the absorbent adopts an expanded state within the bodily cavity.

Fluid may be supplied to the delivery balloon with an electro-pneumatic pump. A vacuum source may also be provided to evacuate fluid from the delivery balloon. The fluid may be a gas.

A method for supplying a therapeutic and/or diagnostic agent to tissue including the step of delivering the therapeutic and/or diagnostic agent to tissue in a pulsing manner by inflating the delivery balloon until said expandable material contacts tissue in the bodily cavity and then deflating the delivery balloon for a desired number of cycles is also provided.

A balloon catheter for supplying a therapeutic and/or diagnostic agent to tissue, comprising a catheter having a distal end and a proximal end; a delivery balloon positioned near the distal end of the catheter, the delivery balloon having an expandable material, being at least partially charged with a therapeutic and/or diagnostic agent, disposed thereon; a proximal balloon positioned proximal of the delivery balloon and a distal balloon positioned distal of the delivery balloon; a first lumen in the catheter through which fluid is supplied to said delivery balloon for inflating the delivery balloon until the expandable material adopts an expanded state against the tissue within a bodily cavity; and at least one additional lumen through which fluid is supplied to the proximal and distal balloons for inflating said proximal and distal balloons, is also provided herein. The proximal and distal balloons, when inflated, create a chamber between the proximal balloon and the distal balloon.

At least one fluid source that supplies fluid to the delivery, proximal and distal balloons may also be provided. The at least one fluid source may further comprise at least one vacuum source that evacuates fluid from the delivery, proximal and distal balloons.

The expandable material may include, but is not limited to, a stent, an absorbent, or an absorbent disposed on at least a portion of a stent. The absorbent may, when cured, adopt a rigid shape within a bodily cavity. In this embodiment, the balloon catheter may also include a probe for applying a curing treatment to the absorbent.

The absorbent may be retained on the delivery balloon in a contracted state with at least one string that is adapted to break when the delivery balloon is inflated.

A balloon catheter for supplying a therapeutic and/or diagnostic agent to tissue, comprising: a catheter having a distal end and a proximal end; a delivery balloon positioned near the distal end of the catheter, the delivery balloon having an expandable material, comprising said absorbent being at least partially charged with a therapeutic and/or diagnostic agent, disposed on at least a portion thereof; and a first lumen in the catheter through which fluid is supplied to the delivery balloon for inflating the delivery balloon until the expandable material adopts an expanded state against the tissue within a bodily cavity is also provided. The absorbent may comprise a sleeve around the delivery balloon.

A method of delivery of a therapeutic and/or diagnostic agent to tissue, comprising the steps of: inserting a catheter assembly into a bodily cavity, the catheter assembly comprising a catheter and a delivery balloon having an expandable material, being at least partially charged with a therapeutic and/or diagnostic agent, disposed thereon, a proximal balloon positioned proximal of the delivery balloon and a distal balloon positioned distal of the delivery balloon, and at least one fluid source that inflates the delivery, proximal and distal balloons by supplying fluid thereto; inflating the proximal and distal balloons by supplying fluid thereto to create a chamber between the proximal balloon and the distal balloon; and delivering the therapeutic and/or diagnostic agent to tissue by inflating the delivery balloon by supplying fluid thereto until the expandable material contacts tissue and is implanted in the bodily cavity.

Other objects of the invention and its particular features and advantages will become more apparent from consideration of the following drawings and accompanying detailed description.

### BRIEF DESCRIPTION OF THE DRAWINGS

FIG. 1 is a side, partial sectional view of a drug delivery system, including a fluid source, a catheter, a balloon and a drug delivery device.

FIG. 2 is a perspective view of a balloon and drug delivery device for use in the drug delivery system of FIG. 1.

FIG. 3 is a side sectional view of a balloon and drug delivery device for use in the drug delivery system of FIG. 1.

FIG. 4 is a cross-sectional view of a catheter for use in the drug delivery system of FIG. 1.

FIG. 5 is a top view of a catheter with an imaging and/or illumination device therein for use in the drug delivery system of FIG. 1.

FIG. 6 is a partially exposed, isometric view of a catheter with an imaging and/or illumination device therein within a bodily cavity for use in the drug delivery system of FIG. 1.

FIG. 7 is a perspective view of a three balloon catheter construct for use in the drug delivery system of FIG. 1.

FIG. 8 is a cross-sectional view of a catheter for use in the drug delivery system of FIG. 1.

FIGS. 9A-9D are partially exposed, side views of the drug delivery system of FIG. 1 with a delivery device being operated within a bodily cavity.

FIG. 10A is a side view of a balloon and drug delivery device for use in the drug delivery system of FIG. 1.

FIG. 10 B is a side view of a balloon and drug delivery device for use in the drug delivery system of FIG. 1.

FIGS. 11A-11C are partially exposed, side views of the drug delivery system of FIG. 1 with a delivery device being operated within a bodily cavity.

FIGS. 12A-12D are partially exposed, side views of the drug delivery system of FIG. 1 having a three balloon catheter construct with a delivery device being operated within a bodily cavity.

FIGS. 13A-13E are partially exposed, side views of the drug delivery system of FIG. 1 having a three balloon catheter construct with a delivery device being operated within a bodily cavity.

### DETAILED DESCRIPTION OF THE INVENTION

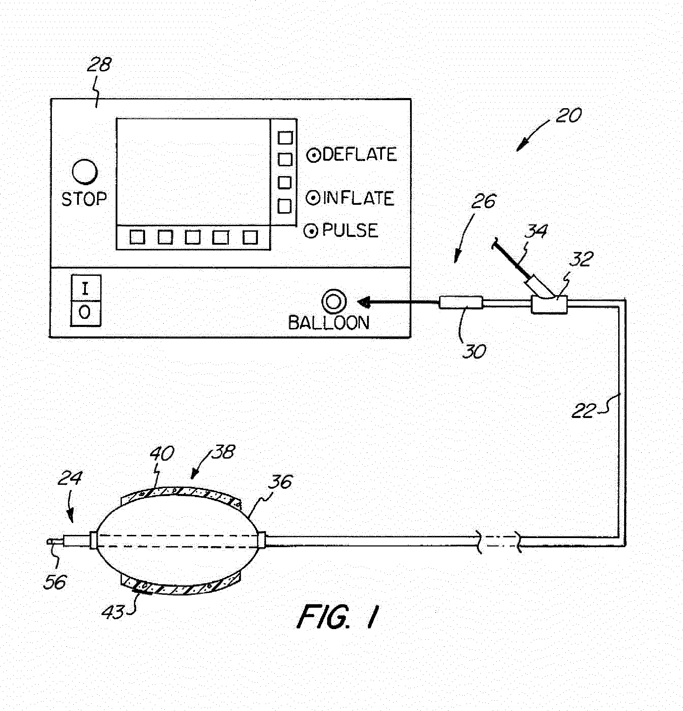

The basic components of one embodiment of an adjustable balloon catheter in accordance with the invention are illustrated in Figure 1. As used in the description, the terms "top," "bottom," "above," "below," "over," "under," "above," "beneath," "on top," "underneath," "up," "down," "upper," "lower," "front," "rear," "back," "forward" and "backward" refer to the objects referenced when in the orientation illustrated in the drawings, which orientation is not necessary for achieving the objects of the invention.

The balloon catheter of the present invention may be used to deliver diagnostic or therapeutic agents to specific locations within a patient's body for diagnostic examination and/or therapeutic treatment. For example, the present invention can be used to deliver drugs, radiation therapies, chemo therapies, pharmacologic medicines, therapeutic agents, immuno-therapies, biologic materials, biologic markers, radiopaque contrasts, diagnostic agents and related technologies to specific cellular locations within and adjacent to bodily cavities, such as tubular structures, lumens, pleural cavities, airways, vessels, organs, bones and joints.

The balloon catheter may be used with guide wires, imaging devices, catheter devices, surgical instruments and tools, operative devices, implants and related medical diagnostic and treatment systems. In an advantageous embodiment, the drug delivery system is used with a resector balloon system described in U.S. Patent Application No. 12/269,495, the disclosure of which is incorporated by reference herein in its entirety.

As shown in FIG. 1, the drug delivery system (20) includes a catheter (22), having a distal end (24) and a proximal end (26), and a fluid source (28). The catheter (22) may have any suitable diameter and length depending on a particular application, and may be flexible, rigid or semi rigid. The catheter (22) may be made with any commercially available material that is flexible enough to allow the shaft to be safely inserted through the available opening of a bodily cavity such that it will bend instead of puncturing the walls of the cavity, and at the same time is rigid enough such as it will maintain its shape as it is passed alongside and/or through the available opening of the bodily cavity. In an advantageous embodiment, the catheter (22) of the drug delivery system (20) consists of a coil wire made of any suitable material, such as stainless steel, and a coating made of polyethylene.

The drug delivery system (20) also includes a delivery balloon (36). The balloon may be made of latex, Yulex, polyethylene, nylon or other suitable material, and may come in a variety of sizes and diameters, which allow the drug delivery system (20) to be used in bodily cavities of various diameters and dimensions, such as large and small bronchial branches, sinuses, and blood vessels, having different types of tumors and tissues to be treated.

Any suitable fluid source (28), such as a manually actuated inflation apparatus or an electro-pneumatic pump, may be used in accordance with the present invention. In the advantageous embodiment shown in FIG. 1, the fluid source (28) is an electro-pneumatic pump having controls on the front thereof, from which a physician or assistant can control the system (as well as a remote control unit), such as that disclosed in U.S. Patent Application No. 2010/0121270 by Gunday et al., the specification of which is hereby incorporated by reference herein in its entirety. A proximal end (26) of the catheter (22) is connected to the pump (28) via a connection mechanism (30). The mechanism (30) may be provided with any suitable connector or a plurality of connectors, such as a luer connector, for connection to the manually actuated expansion apparatus or inflation pump (28).

The manually actuated inflation apparatus and/or pump (28) supplies a fluid, such as a gas, liquid, or mixture thereof, to the delivery balloon (36) via a lumen, or a plurality of lumens provided in the elongated catheter shaft (22). The pump (28) also includes a variety of capabilities for balloon identification, proper inflation/deflation of the balloons, and feedback measurements, many details of which are described in Gunday et al. In certain advantageous embodiments, the pump (28) further includes a vacuum source to evacuate fluid from the catheter (22). Any suitable type of a manually actuated inflation apparatus or pump may be used in accordance with the present invention. In one advantageous embodiment, a PhysioSense pump is used, which may control the amount of inflation, expansion and/or activation of the expansion apparatus based on actual bodily lumen measurements and confirmed by pressure reading as well to ensure appropriate expansion.

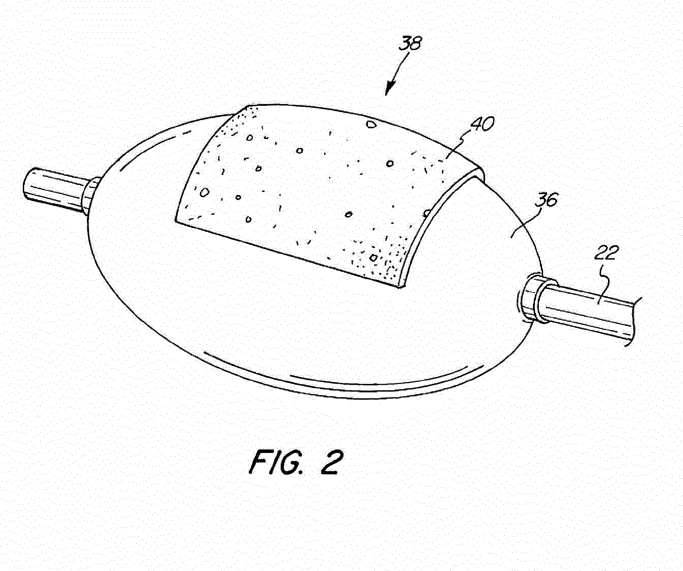

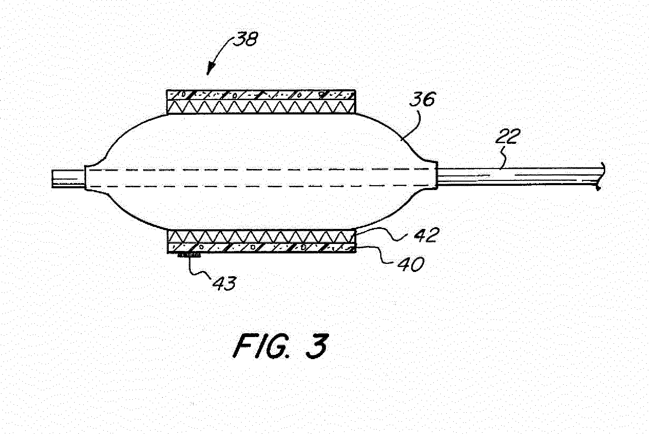

A delivery device (38) is disposed on at least a portion of the delivery balloon (36). In an advantageous embodiment, the delivery device (38) is made of an expandable absorbent (40). The absorbent may be a sponge-like material such as a stretchable textile material. The absorbent (40) may be formed as a sleeve encircling the delivery balloon (36), as shown in FIG. 1, or it may be only partially disposed on a surface of the delivery balloon (36) like a patch, as shown in FIG. 2. The delivery device (38) may also be a stent (42), as shown in FIG. 3. In one advantageous embodiment, a layer of expandable absorbent (40) is at least partially disposed on the outer surface of the stent (42). The delivery device (38) may also be biodegradable.

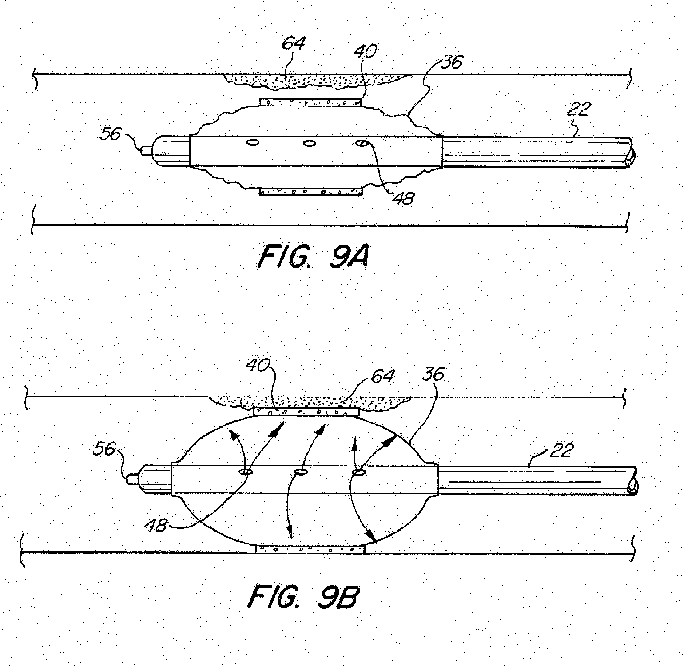

The absorbent (40) may be capable of being cured to adopt a particular shape by applying pressure, ultraviolet light, infrared light, thermal energy, ultrasound, radiofrequency waves, electrical current, or electrical field. As illustrated in FIGS. 9A-9D, the absorbent (40) of the delivery device (38) may be cured to adopt a rigid shape corresponding to the shape of the inner surface of a body lumen within a patient's body.

Selected parts and/or the entirety of the delivery device (38) may be charged or impregnated with the therapeutic and/or diagnostic agent to be delivered to the target tissue. The delivery device (38) can be pre-prepared, such as by soaking the absorbent (40) prior to the procedure, or it can be prepared in the operating room. Alternatively, portions of the delivery device (38) may be selectively charged with the therapeutic and/or diagnostic agent, such as by using a dropper. The delivery device (38) can be charged with the therapeutic and/or diagnostic agent only in the areas specific to the tissue that is targeted. The delivery device (38) can also be multi-layered where each layer contains different drug or has different drug migration timing which can be activated by pressure, light (UV, IR, etc.), heat, cold, ultrasound, RF, electrical current/field and other means.

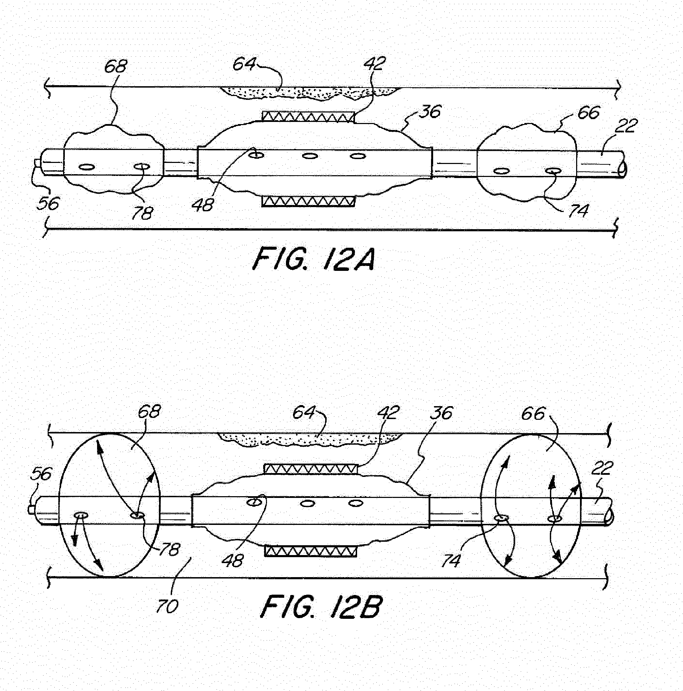

The expandable delivery device (38) may be releasably disposed on the outer surface of the delivery balloon (36) so that it may be implanted into and left in a patient's body to achieve prolonged infusion of the diagnostic and/or therapeutic agent. For example, where the delivery device (38) is a stent (42), as will be further explained with respect to FIGS. 12A-12D, the stent (42) is releasably disposed on the delivery balloon (36) so that it may be separated from the balloon once deployed in a radially extended state and implanted within the bodily cavity. However, it is also contemplated that the expandable delivery device (38) may be rigorously affixed to the outer surface of the delivery balloon (36) so as to remain on the delivery balloon (36) and be removed from the body after use.

In an advantageous embodiment, the delivery balloon (36) has a wall with outer surface that comprises an abrasive material intended to abrade bodily tissues, such as airway or vessel walls. The abrasion of the bodily tissues stimulates bleeding and instigates flow of white blood cells, i.e. leukocytes, out of the circulatory system towards the site of tissue damage. This process, together with the application of volumetric pressure or force to the abraded surface of the airway or the vessel wall to neutralize hemodynamic shear forces, perpetuates fluid extravasation processes and stimulates associated cellular absorption of the diagnostic and/or therapeutic agents into the adjacent tissues.

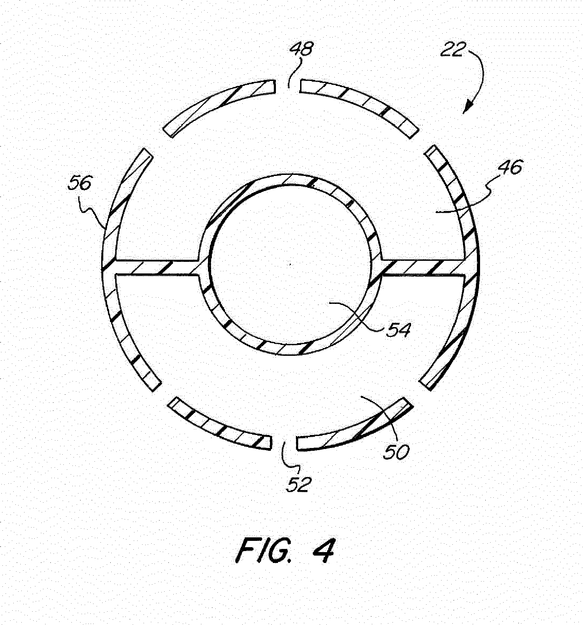

FIG. 4 illustrates a cross section of the catheter (22) of the drug delivery system (20). Catheter (22) includes at least a first lumen (46). The first lumen (46) is in fluid communication with the delivery balloon (36) via at least one opening (48) in outer wall (56) and is used to supply fluid from the fluid source (28) to inflate the delivery balloon (36). A second lumen (50) may also be provided to deliver a therapeutic, diagnostic and/or imaging agent into the bodily cavity via at least one opening (52). The at least one opening (48, 52) is intended to cyclically deliver and evacuate the agents and various other fluids instantly, sequentially, intermittently and/or constantly over designated time intervals.

The catheter (22) further includes a center lumen (54), which can be used to deliver any number of things to assist insertion and positioning of the drug delivery system (20) within the bodily cavity and to carry out various medical procedures. For example, center lumen (54) may be used as a conduit for a guide wire (58) when inserting the delivery balloon (35) into the bodily cavity in a deflated state. The center lumen (54) may extend the entire length of the catheter (22). The center lumen (54) can also be used as a bypass channel to allow bodily fluids, such as air or blood, to flow through the balloon catheter, which is necessary in certain medical applications, e.g. pulmonology or cardiology. In this example, the center lumen (54) of the catheter (22) extends through the catheter tip and is open at the distal end (24).

It should be noted that additional lumens may be provided in the catheter (22) for introduction of various medical instruments to carry out various diagnostic or therapeutic procedures, or as will be presented below, to inflate additional balloons. For example, additional lumens may be provided for introduction of an imaging and/or illumination system to facilitate the movement of the catheter construct through the cavities and to assure precise positioning of the guide wire (58) at the target site.

As shown in FIG. 1, in an advantageous embodiment, the catheter (22) also includes a connection port (32) for insertion of an imaging and/or illumination device (34) or other probe. The connection port (32) may be a lumen breakout V junction to facilitate the introduction of the imaging and/or illumination device (34) or other probe. The connection port (32) may also be a Y junction to bring out a separate, inner or auxiliary lumen (not shown) of the catheter (22) to a suitable connector. Additionally, the connection port (32) could also include a shut-off valve for stopping the delivery balloon (36) from deflating.

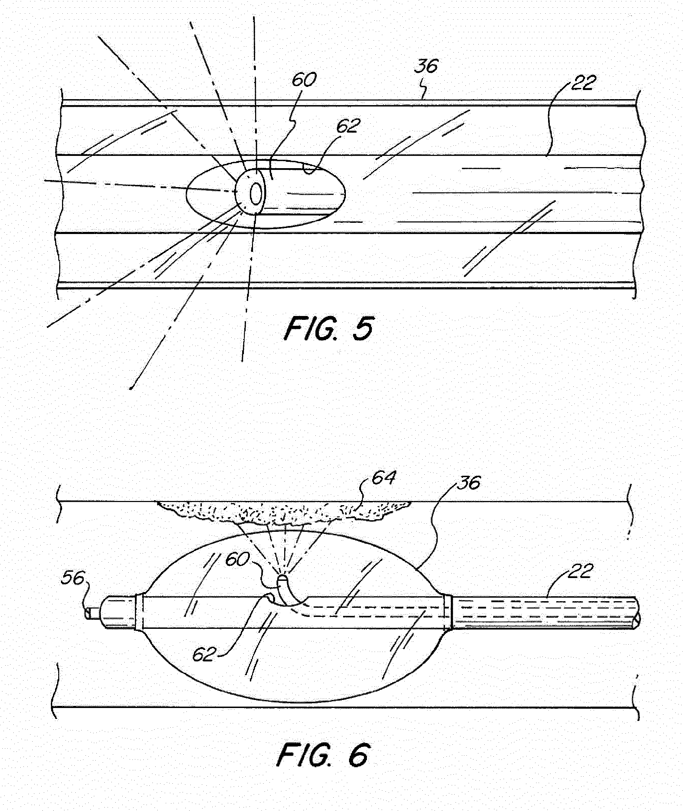

Accordingly, an imaging and/or illumination device (34) may also be employed with the drug delivery system (20). In some embodiments, as shown in FIGS. 5 and 6, a fiber optic image bundle (60) is introduced through the center lumen (54) of catheter (22) or any inflation or auxiliary lumen (not shown) via port (32) to image the surrounding area. The fiber optic image bundle (60) can be made of an incoherent fiber bundle for illumination and a coherent imaging fiber bundle at the core, and a lens. The bundle (60 may incorporate various types of object lenses at the distal tip for different field of view (i.e. 50°, 130°, etc.) and various depth of field. Two separate bundles, one for illumination and the other for image can also be used. At the distal end of the fiber optic bundle (60), the imaging coherent fibers are separated from illumination fibers (not shown) and interfaced to an image sensor, such as CMOS or CCD, through appropriate optics (not shown). Similarly, the illumination fibers are interfaced to a light source (not shown). It should be noted, however, that other sources of illumination, such as light emitting diodes, may also be employed. It should also be noted that the image sensor (CCD or CMOS available today in 2mm size) can be located at the tip of the imaging catheter assembly (not shown), eliminating the need for coherent imaging fiber bundle, thus increasing the image quality and reducing cost.

The imaging and/or illumination device (34) provides the physician or user with illuminated light, non-thermal illuminated light, and direct visual feedback of the area ahead of the delivery balloon (36), along the sides of the balloon, and/or behind the balloon. As illustrated in FIG. 6, it is particularly advantageous for the catheter and/or the balloon to be made of a transparent or translucent (i.e., see-through) material so that the bundle (60) can provide images of the surrounding bodily cavity while it is maintained within the catheter (22) or within the delivery balloon (36). The transparent or translucent balloon allows the bundle (60) to provide imaging and/or illumination capabilities that facilitate positioning the drug delivery system (20). As shown in FIGS. 5 and 6, the catheter (22) may contain an opening (62) through the outer wall (56) of the catheter (22) within the balloon (24) such that the bundle (60) can pass through the opening (62) into the delivery balloon (36) for more direct imaging or lighting of a target tissue (64). The imaging sensor and illumination optics possess the ability to be translated linearly or rotationally through and/or around the balloon (36), thereby allowing for 360° visualization of the treatment area.

In some advantageous embodiments, the distal end (24) of the catheter (22) includes a transparent membrane made out of any suitable material. The imaging and/or illumination device (34) is extended through one of the lumens of the catheter (22) to the membrane, which allows for visualization of the area ahead of the distal end (24) of the catheter (22). In this way, the physician can be provided with illuminated light and direct visual feedback of the area ahead of the balloon catheter, along the sides of the balloons, and/or behind the balloons.

In other advantageous embodiments, the lumen of the catheter (22), in which the imaging device is disposed, has an opening at a distal end, and the imaging device is extended out of the opening to visualize tissue in front of the drug delivery device (20). In this embodiment, the catheter (22) can also be provided with a cleaning device (not shown) at the distal tip for cleaning the imaging and/or illumination device (34). The cleaning device is made with any suitable type of material, such as textile bundle, and is affixed to an inner surface of the catheter (22) adjacent to the opening at the distal end. The imaging device is cleaned by moving it back and forth through the textile bundle, thus wiping a lens of the imaging device.

In cases where holes (e.g. 62) are to be used or it is desirable to look back, the imaging devices come with a pre-shaped distal tip. This feature enables the distal tip to come out of the side holes (with the rotation of the image guide outside the body when the distal tip reaches the hole). If a three balloon catheter is deployed (explained below) using a flexible or rigid endoscope, then the imaging from the endoscope is also available at the proximal end.

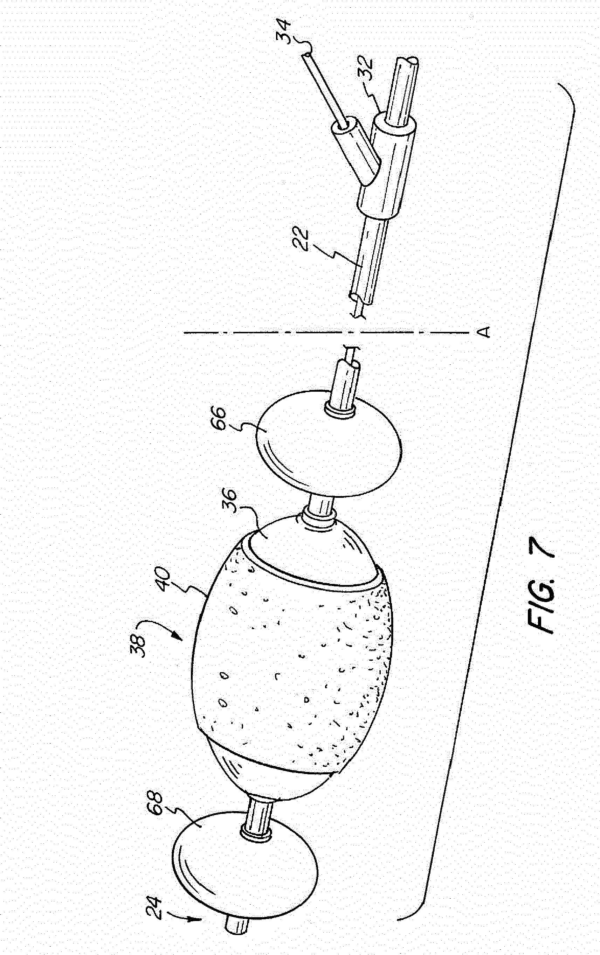

In an advantageous embodiment, shown in FIG. 7, the drug delivery system (20) also includes a proximal balloon (66), located proximal of the delivery balloon (30) along the catheter (22), and a distal balloon (68), located distal of the delivery balloon (30) along the catheter (22). The proximal (66) and distal (68) balloons may be made of latex or other suitable material, and may come in a variety of sizes and diameters, which allow the adjustable balloon catheter to be used in bodily cavities of various diameters and dimensions.

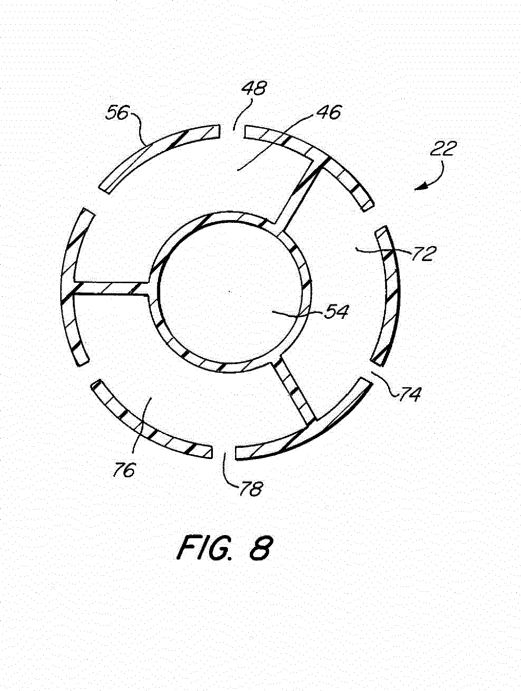

As illustrated in FIG. 8, in the advantageous embodiment having a three balloon construct, the catheter includes a first lumen (46) to supply fluid from the fluid source (28) to inflate the delivery balloon (36) via at least one opening (48) in the outer surface (56) of the catheter (22). Second (72) and third (76) lumens may also be provided in the catheter (22). The second (72) and third (76) lumens are in fluid communication with the proximal (66) and distal (68) balloons via openings (74) and (78) and are used to supply fluid from the fluid source (28) to inflate the proximal (66) and distal (68) balloons, respectively. Catheter (22) also includes a center lumen (54).

The drug delivery system (20) may include a plurality of openings along the catheter (22) between the proximal (66) and distal (68) balloons and/or emanating out of the surface membrane of the balloons. In certain embodiments, the openings are intended to deliver the diagnostic and/or therapeutic agents. In certain other embodiments the openings are intended to evacuate the agents and/or other fluids that may be present at the site or from within the chamber (70) using negative pressure via suction/vacuum. In various other embodiments the openings are intended to cyclically deliver and evacuate the agents and various other fluids instantly, sequentially, intermittently and/or constantly over designated time intervals.

The delivery (36), proximal (66) and distal (68) balloons of the drug delivery system (20) may advantageously have a textured outer surface which acts as a gripping surface for attachment to bodily tissues, such as blood vessel walls, to anchor the delivery balloon (36) and/or the proximal (66) and distal (68) balloons at a target tissue site. In some embodiments, the surface of the delivery (36), proximal (66) and distal (68) balloons may comprise a fiber mesh affixed to the surface of the balloon during the molding process, which produces outwardly-facing protrusions on the surface of the balloon that assist in gripping of the balloon to the surrounding tissue. The fiber mesh may be made of lycra, polyurethane, composite springs, or other appropriate material. In other embodiments, dimensional surface structures or inflatable sinuses that are encapsulated in the surface substrate of the balloon may be used to produce the surface protrusions.

Accordingly, the drug delivery system (20) having a three balloon construct is particularly advantageous. The proximal (66) and distal (68) balloons may grip the inner surface of the bodily cavity to provide stabilization and prevent migration of the system (20) during implantation or pulsation of the delivery device (38). Moreover, the proximal (66) and distal (68) balloons may abrade the bodily cavity to enhance infusion of the diagnostic and/or therapeutic agents into the target tissue.

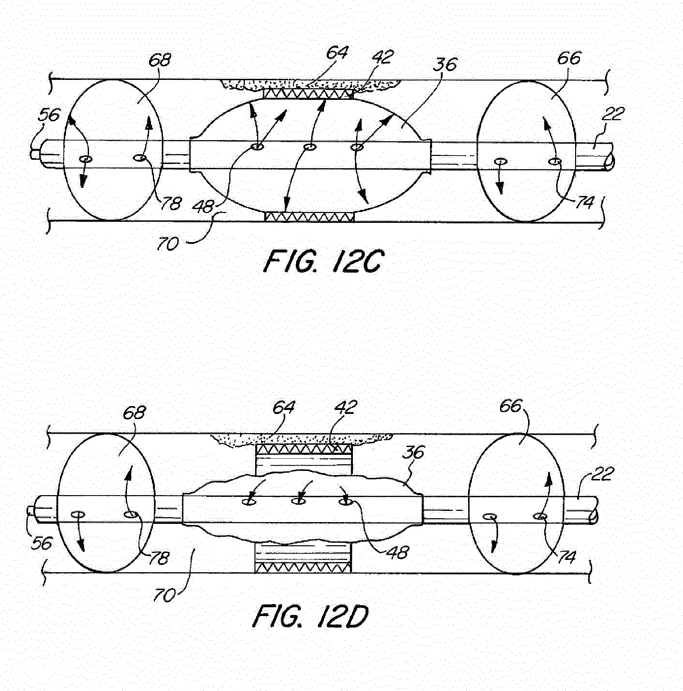

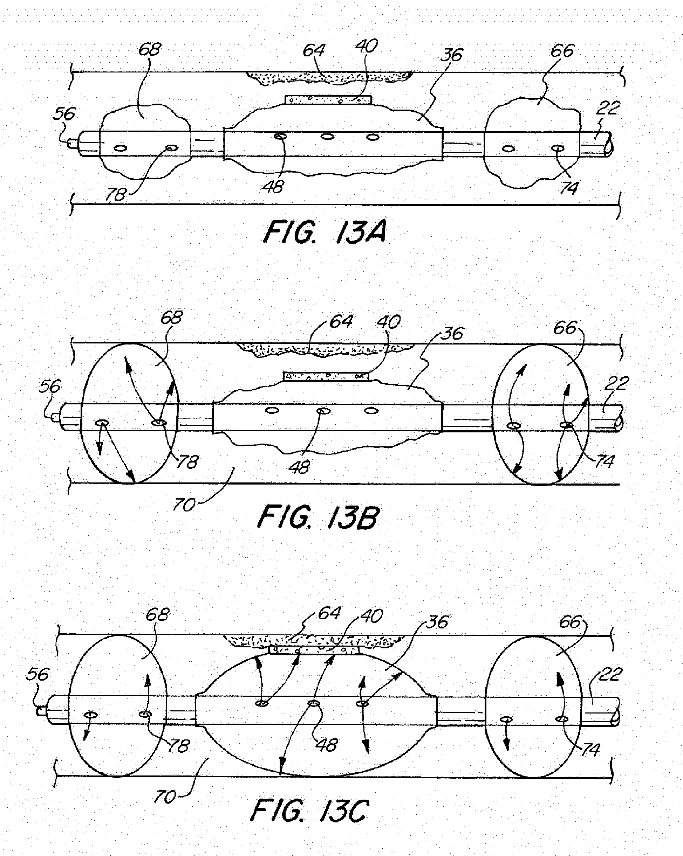

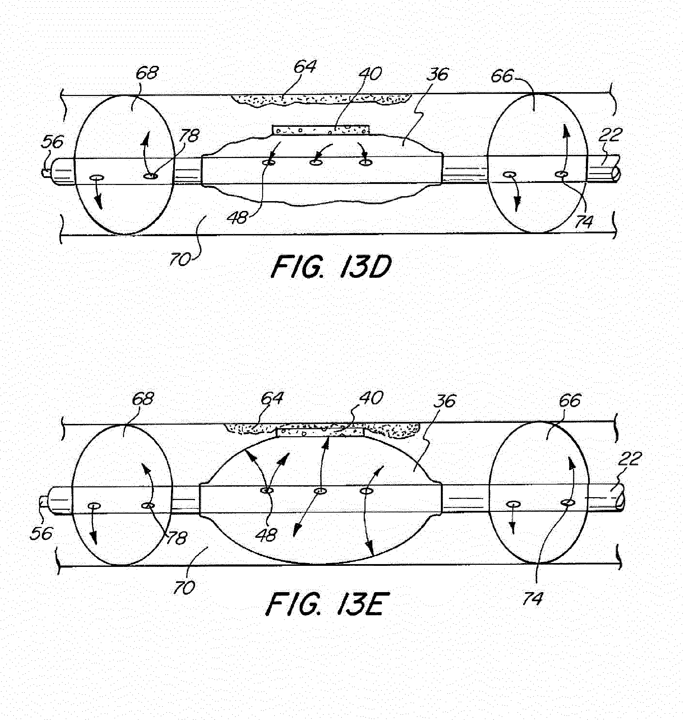

Further, as shown in FIGS. 12 and 13, the proximal (66) and distal (68) balloons create a chamber (70) therebetween when inflated. Various diagnostic and/or therapeutic agents, such as drugs, radiation therapies, chemo therapies, pharmacologic medicines, therapeutic agents, immuno-therapies, biologic materials, biologic markers, radiopaque contrasts, diagnostic agents and related technologies may be delivered and/or removed from the chamber (70), instantly or cyclically, through the openings in the lumen walls of the catheter construct and/or through openings on the membranes of the expansion apparatus to affect extravasated drug delivery to specific locations within the bodily cavity. This localization of any agents used during a procedure acts to prevent infusion into unwanted tissues. For example, if imaging and/or contrast agents are utilized, the chamber may be lavaged and evacuated before removal of the system (20) to prevent the agents from escaping into the surrounding tissue.

The operation of a drug delivery system (20) can generally be described with reference to FIGS. 9A-9D. Referring first to FIG. 9A, after a visual inspection via an endoscope, x-ray, and/or ultrasound, a drug delivery system (20) is selected. As explained above, the delivery device of the drug delivery system (20) may be pre-charged with the desired therapeutic and/or diagnostic agent(s), or it may be prepared in the operation room. The deflated delivery system (20) is then inserted into position in a bodily cavity. This may be accomplished by using the working channel of an endoscope or, as previously noted, along a guide wire (56) that is previously inserted into the body and inserting the proximal end of the guide wire (56) which is outside the body into the center lumen (54) of the catheter. The catheter (22) is then advanced over the guide wire.

The appropriate positioning of the drug delivery system (20) may be determined by aligning markings on the distal and/or proximal end of the guide wire with the distal and/or proximal end of the catheter's center lumen (54). The step may be monitored under fluoroscopy to ensure that the balloon or expansion apparatus has not moved out of position. An imaging system may also be employed during this procedure through inner channels provided in the catheter.

In one advantageous embodiment, the drug delivery system (20) is introduced into the body via a steerable catheter having an imaging system. In other embodiments, a catheter system with radio opaque markers may be used for indirect imaging.

Referring next to FIG. 9B, the delivery device (38), in this case a sleeve of absorbent (40), such as a sponge-like material, disposed on the delivery balloon (36) is positioned near target tissue (64) within the bodily cavity. It is advantageous to use imaging means to facilitate this step, as described above. Simultaneously, or after positioning the delivery device (38), fluid is supplied to the delivery balloon (36) via the first lumen (46) in the catheter (22) through the at least one opening (48) to inflate the balloon (36). It should be noted that, although three openings in the catheter (22) are illustrated in FIGS. 9A-9D, one opening is sufficient to supply fluid to inflate the delivery balloon (36).

As the delivery balloon (36) inflates, delivery device comes in contact with the target tissue (64). In this embodiment, the sleeve of absorbent (40) is designed such that, in an expanded state, it expands to conform to the diameter and general shape of the body lumen. Once in contact with the target tissue (64), the diagnostic and/or therapeutic agent(s) will begin to infuse into the target tissue (64) from the absorbent (40). The delivery balloon (36) may be inflated further to squeeze the absorbent between the balloon (36) and the wall of the bodily cavity to urge the agent into the tissue. As explained above, migration of the diagnostic and/or therapeutic agent(s) from the delivery device into the target tissue (64) may be activated, monitored and/or modified by the application of pressure, light (UV, IR, etc.), thermal energy, ultrasound, radio frequency waves, electrical current, electrical field, etc.

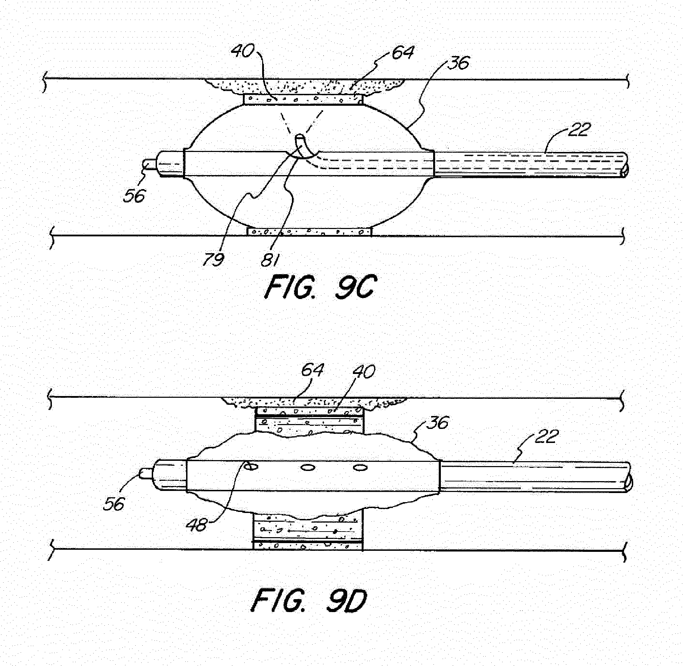

Once, the delivery device (38) is appropriately positioned and pressed against the target tissue (64), the sleeve of absorbent (40) may be cured to adopt a rigid shape within and conforming to the shape of the bodily cavity. As shown in FIG. 9C, a probe (79) may be introduced into the central lumen (54) of the catheter (22) until it reaches the area of the delivery device. The probe (79) may apply pressure, ultraviolet light, infrared light, thermal energy, ultrasound, radiofrequency waves, electrical current, or electrical field, etc., to the absorbent (40) to achieve curing of the material. The probe (79) may be inserted into the catheter construct similar to the imaging/illumination device discussed with reference to FIGS. 5 and 6. An opening (81) may be provided in the catheter to allow the probe to extend therethrough. Alternatively, the probe may apply the curing treatment through the catheter (22) and balloon (36).

After curing of the absorbent (40) is achieved and the delivery device (38) is implanted within the bodily cavity, the delivery balloon (36) is deflated by evacuating fluid via the first lumen (46) in the catheter (22) through the at least one opening (48). The catheter (22) is then removed from the bodily cavity. Because the absorbent (40) was releasably disposed on the delivery balloon (36), the delivery device (38) remains implanted within the bodily cavity even when the delivery balloon (36) is deflated as shown in FIG. 9D. This allows for prolonged contact of the absorbent (40) with the target tissue (64) and, thus, greater infusion of the diagnostic and/or therapeutic agent without prolonged inflation of the delivery balloon (36) within the bodily cavity.

In certain advantageous embodiments, after the expandable delivery device (38) has been implanted in the tissue at the target cite, the delivery balloon (36) is deflated and the entire catheter (22) is removed from the bodily cavity. After a certain period of time, once at least a portion of the diagnostic and/or therapeutic agent has been delivered to the target tissue, it may be desired to return to the target cite and recharge the delivery device (38) with additional agent so that additional investigation, diagnosis and/or treatment may be administered. This recharging may be achieved by introducing an agent-delivering probe into the bodily cavity at the location of the delivery device (38).

The expandable delivery device (38) may also include a micro RF tag transmitter (43) that enables locating the implanted delivery device (38) and identifies its type, the therapeutic and/or diagnostic agent(s) that was used and the date it was implanted. The receiving and activation antenna for RF tag (43) may be included in the catheter and may be electrically connected to a controller outside the body. In this advantageous embodiment, the surgeon may implant a delivery device having an RF tag (43) and easily return to the target site at some later time after some amount of the diagnostic and/or therapeutic agent has been delivered to the tissue to recharge the absorbent (40) with additional agent.

Any of the various agents useful in therapeutic application can be delivered in the above described manner. For example, the agent may comprise one or more chemical or biological drugs with useful pharmacological properties, as well as any other medicaments or other substances with medicinal or other therapeutic uses. Such agents may be synthetic or natural, so long as they have an advantageous therapeutic effect that can be obtained by delivering the agent to a target site. In certain embodiments, agents particularly useful for chemotherapies, radiation therapies, or immunotherapies are delivered as described above.

In some advantageous embodiments, a cytotoxic substance or other agent useful for chemotherapy is delivered to a target site via the balloon catheter system with delivery probe of the present invention. For example, in some cases, the catheter system is used to deliver a chemical agent that affects cell division or DNA synthesis. Such agents include, for example, alkylating antineoplastic agents, such as cisplatin, carboplatin, oxaliplatin, mechlorethamine, carmustine, cyclophosphamide, chlorambucil, ifosfamide, busulfan, treosulfan, melphalan hydrochloride, thiotepa, and dacarbazine; anti-metabolites, such as azathioprine, mercaptopurine, thioguanine, fludarabine, pentostatin, cladribine, fluorouracil, floxuridine, cytosine arabinoside, gemcitabine, methotrexate, pemetrexed, and raltitrexed; anthracenedione antineoplastic agents, such as mitoxantrone; anthracyclines, such dactinomycin, daunorubicin, doxorubicin, epirubicin, idarubicin, valrubicin, aclarubicin, and bleomycin; plant alkaloids and terpenoids, such as noscapine, vincristine, vinblastine, vinorelbine, vindesine, podophyllotoxin, paclitaxel, and docetaxel; topoisomerase inhibitors, such as irinotecan, topotecan, amsacrine, etoposide, etoposide phosphate, and teniposide; and other agents with similar mechanisms of action, such as mitomycin C.

Other such agents include those that target molecular abnormalities, including tyrosine kinase inhibitors, such as crizotinib, gefitinib, erlotinib hydrochloride, imatinib, and imatinib mesilate. Still other such agents include those that modulate tumor cell behavior without actually attacking the cells, such as may be employed for hormone treatments. Indeed, any drug known to be efficacious in treating cancerous cells, such as streptozotocin or diltiazem augment taxol, may be employed.

In certain advantageous embodiments, a biological response modifier or other biological agent useful for immunotherapy is delivered to a target site via the balloon catheter with delivery probe. Such agents, which are often cytokines, may be a recombinant, synthetic, or natural preparation. These biological agents may include, for example, interferons, such as alpha-interferons and beta-interferons; interleukins, such as aldesleukin; colony-stimulating factors, such as filgrastim, sargramostim, epoetin, and oprelvekin; monoclonal antibodies, such as edrecolomab, rituximab, trastuzemab, gemtuzumab, alemtuzumab, nimotuzumab, cetuximab, bevacizumab, ibritumomab, panitumumab, and tositumomab; cancer vaccines; gene therapies; and non-specific immunomodulating agents. Any biologic known to useful for immunotherapies, such as asparaginase, may be employed.

In some advantageous embodiments, the therapeutic agent is delivered in drug eluting microspheres, which can be used both to cause the embolization of blood vessels that supply undesirable tissues and to retain the drug in a localized area for a sustained period of time. For example, drug-eluting microspheres can be used to deliver a chemotherapeutic drug, such as doxorubicin, to a tumor. When the microspheres reach the target site, they will block vessels supplying the tumor, and this suppression of blood flow will lead to ischemia. Over time, the microspheres break down, and the drug will be absorbed by the tissue. As a result, not only is a localized sustained realease of the drug achieved, but the ischemia will also increase the effect of the drug on the tumor.

The above described delivery of therapeutic agents is also useful for radiation therapies, in which high-energy radiation is used to kill cancer cells and shrink tumors. One method of such therapy places radioactive material in the body near the cancer cells. Thus, in certain advantageous embodiments, a radioactive substance, such as a radiolabeled monoclonal antibody, is supplied via the balloon catheter with delivery probe and extravasated into nearby tissue as described below.

Various agents may also be employed to assist in making diagnostic observations or monitoring procedures. For example, in some advantageous embodiments, the above described system may be used to deliver a contrast agent that allows or improves visualization via one or imaging modalities, which can be used to image the extravasation of the agent into the surrounding tissues throughout the course of a procedure. Such agents may include, for example, radiocontrast agents, such as iodine or barium, to improve X-ray based imaging techniques; MRI contrast agents, such as gadolinium, to improve magnetic resonance imaging; and microbubble contrast agents, to improve ultrasound imaging.

In some advantageous embodiments, biomarkers are used together with a therapeutic agent to observe and monitor the extravasation of the agent into the surrounding tissues. In some of these advantageous embodiments, CF3PM & MTFN-1 fluorinated radio-opaque biomarkers are used. The biomarkers may be detected by various non-invasive imaging modalities, such as X-Ray, MRI, CT, ultrasound, spectroscopy, etc.

With the addition of an appropriate inert dye or contrast media (radioactive, polarized, florescent, temperature sensitive, etc.), along with the diagnostic and/or therapeutic agent, the infusion rate and the amount of infusion into the tissue can be monitored, quantified, displayed and reported by a controller by capturing sequential video frames under different illumination conditions (UV, IR, polarized, color filters, etc.). This information may be used to visually identify the extravasation depths and/or discern the requisite volumetric pressure, force, temperature, frequency and/or time to achieve efficacious delivery of the agent to desired depths of penetration at the intended treatment site. The drug delivery system (20) may also consist of radiopaque markings and calibrations that aid direct visualization via endoscopic imaging and in-direct visualization via Radiography, MRI, CT, Ultrasound and/or other modalities of imaging known in the art. Once the agent or contrast media has reached, and sufficiently saturated the intended treatment site, the remaining agent or contrast media is removed. The body lumen is then irrigated, lavaged and suctioned to remove all remaining agent or contrast media.

Further, any of the above-mentioned diagnostic, therapeutic and/or imaging agents may be provided within polymer microspheres that may be dispersed within the delivery device (38). The microspheres may be designed to disperse the agents contained within upon the application of pressure, light (UV, IR, etc.), thermal energy, ultrasound, radio frequency waves, electrical current, electrical field, etc. Alternatively or additionally, the microspheres may be biodegradable. In one advantageous embodiment, the microspheres may be sticky or coated with a sticky material so that they will stick to the target tissue(s) within a bodily cavity.

The drug delivery system (20) of the present invention can also be used to supply various media, e.g. light based therapies, radiofrequency wave forms, thermal energies and temperatures, and pressured air, to modulate cellular response sufficient to achieve tumoral destruction and to alter cellular membrane integrity to facilitate extravasation of medicinal and/or diagnostic agents into bodily tissues.

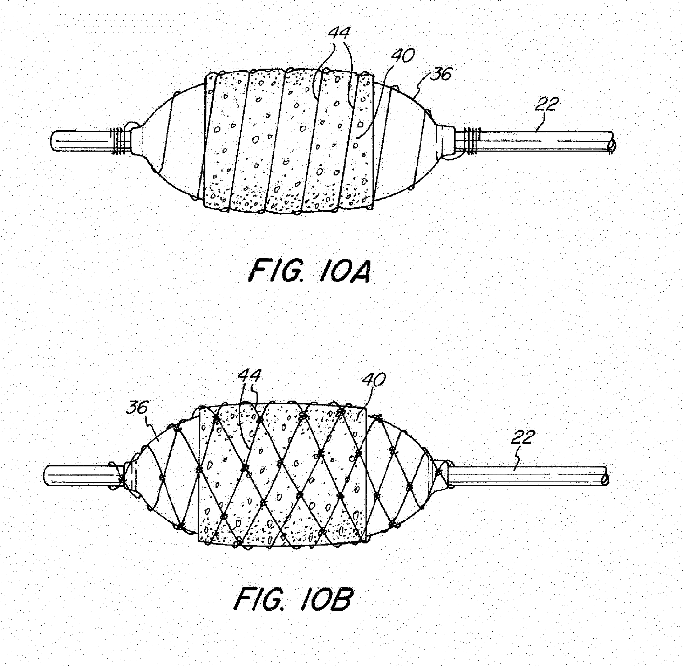

In another embodiment of the drug delivery system (20) of the present invention shown in FIG. 10A-B, the expandable delivery device (38) comprises a sleeve of absorbent (40) that is held on the delivery balloon in a contracted state by a string (44). The string may be attached to the catheter (22) at either side of the delivery balloon (36) and may be wound or woven around the delivery device (38), such as to form a net. The string may be made of any suitable material selected so that it will break once put under tension by the expanding delivery balloon (36). Alternatively, the string may be pre-fabricated to have one or more weakened areas so that the string is predisposed to break at those points. The string (44) may alternatively be designed to detach from one side of the catheter (22) upon expansion of the delivery balloon (36).

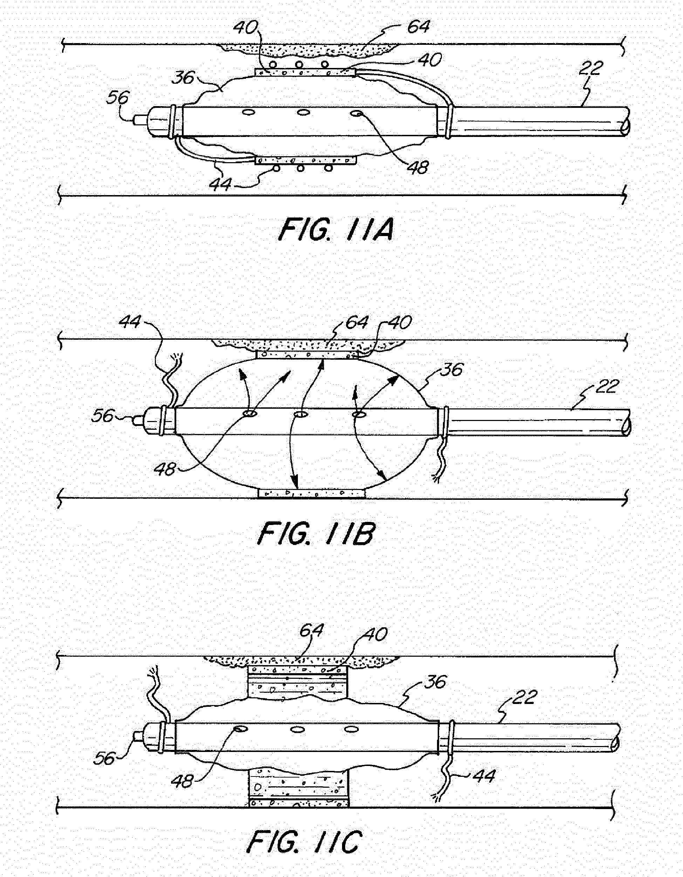

The operation of this embodiment of a drug delivery system (20) is illustrated in FIGS. 11A-11C. The drug delivery system (20) is prepared and is introduced into a bodily cavity and positioned near target tissue (64) as explained above with respect to FIGS. 9A-9D. When delivery balloon (36) is expanded, string (44) is put under tension until it breaks or otherwise separates from the catheter (22). No longer constrained by the string, the sleeve of absorbent (40) is permitted to adopt an expanded shape, much like a spring. Once the absorbent (40) is deployed into its expanded state, the delivery balloon (36) may be inflated further to squeeze the absorbent between the balloon (36) and the wall of the bodily cavity to urge the agent into the tissue. The delivery balloon (36) is then deflated and removed from the body as shown in FIG. 11C.

In this embodiment, the absorbent (40) is resilient in nature such that it will readily return to an expanded state after being deformed by the string (44). Moreover, the sleeve of absorbent (40) is sized such that, in an expanded state, it will expand to conform to the diameter and general shape of the body lumen. The surface of the delivery device (38) may also be ribbed, may have protrusions or spikes, or may generally be rough such to aid in gripping the inner surface of the body lumen to prevent migration of the absorbent (40).

An advantageous method of operation, employing a drug delivery system (20) having a three balloon catheter can generally be described with reference to FIGS. 12A-12D. As described above, the delivery device (38) may be pre-charged with the diagnostic and/or therapeutic agent or it may be prepared while in the surgery room. Moreover, the stent (42) may also be provided with a sleeve of absorbent (40), such as a sponge-like material, that may be wholly or partially charged with a diagnostic and/or therapeutic agent. While FIGS. 12A-12D illustrate the use of a stent (42) as the delivery device (38), it should be noted that any of the agents and delivery devices (38) described herein may advantageously be used with the three balloon catheter system.

Referring first to FIG. 12A, after a visual inspection via an endoscope, x-ray, and/or ultrasound, a drug delivery system (20) having a catheter with at least three balloons is selected, and the system (20) is inserted into a bodily cavity until the delivery device (38) disposed on a delivery balloon (42) is positioned near a target tissue (64). This may be accomplished by using the working channel of an endoscope or, as previously noted, along a guide wire (56). The delivery system (20) is connected to a pump, at which time the pump may determine the type of balloon catheter (22) that has been inserted.

Referring next to FIG. 12B, fluid is supplied to the proximal (66) and distal (68) balloons through via the inflation lumens (72, 76) and through the at least one openings (74, 78) to inflate the balloons (66, 68), until the balloons are in sufficient contact with the bodily cavity to seal the target tissue (64) within a delivery chamber (70). After the proximal (66) and distal (68) balloons are inflated, fluid is supplied to the delivery balloon (36) via the first lumen (46) in the catheter (22) through the at least one opening (48) to inflate the balloon (36).

In the case of the delivery device (38) as a stent (42), delivery balloon (36) is inflated until the stent "snaps" into its radial extended state and is implanted into the body lumen. In this state, the pressure of the stent against the target tissue will cause the diagnostic and/or therapeutic agent to infuse into the target tissue (64). As explained above, the delivery balloon (36) may be inflated further to squeeze the absorbent between the balloon (36) and the wall of the bodily cavity to urge the agent into the tissue. Migration of the diagnostic and/or therapeutic agent(s) from the delivery device (38) into the target tissue (64) may be activated, monitored and/or modified by the application of pressure, light (UV, IR, etc.), thermal energy, ultrasound, radio frequency waves, electrical current, electrical field, etc. The stent (42) may advantageously be specially designed for easy removal and can be removed with a specially designed instrument. Thus the procedure can be repeated as needed.

Once the stent (42) is implanted, the delivery balloon (36) may be deflated and additional diagnostic, therapeutic and/or imaging agent(s) may then be delivered and released within the chamber (70) from delivery lumens therein and onto surfaces of the surrounding bodily cavity and target tissue (64). Optionally, as described below, when a pulse button on the pump is pressed, the delivery balloon (36) is deflated and inflated in a cyclical fashion. Proximal (66) and distal (68) balloons remain inflated, preventing the diagnostic and/or therapeutic agent from spreading outside the delivery chamber (70).

The drug delivery system (20) having a three balloon catheter may further include inline pressure valves that enable the lumens (46, 72, 76) to be pressurized, while preventing unintended de-pressurization. The delivery chamber (70) is pressurized by inflating the delivery balloon (36) and/or by pressurizing the chamber (70) with pneumatic pressured air or by other means. In an advantageous embodiment the diagnostic and/or therapeutic agent contains a radiopaque contrast that permits visualization such as, Radiography, MRI, CT, Ultrasound, Endoscopic and/or other means of visualization known in the art to image the extravasation of the agent into the surrounding tissues throughout the course of treatment. Once the agent has reached, and sufficiently saturated, the intended treatment site with localized delivery of the agent, the remaining agent is removed. The chamber is then irrigated, lavaged and suctioned to remove all remnant agent. The proximal balloons (66, 68) are deflated, contracted and/or de-activated and the construct is pulled out of the body.

In a further advantageous embodiment of the drug delivery system (20) of the present invention shown in FIGS. 13A-13E, the expandable delivery device (38) comprises a patch of absorbent (40) disposed on a portion of the delivery balloon (36). Preferably, the absorbent (40) is rigorously attached to the outer surface of delivery balloon (36) so that it will not detach from the balloon while in the bodily cavity. The absorbent (40) may be prepared and charged with a diagnostic and/or therapeutic agent as discussed above. The system (20) is inserted into a bodily cavity until the delivery device (38) disposed on a delivery balloon (42) is positioned near a target tissue (64).

If, as shown in FIGS. 13A-13E, a three-balloon construct is chosen, the proximal (66) and distal (68) balloons are inflated. The delivery balloon (36) is then inflated until the patch of absorbent (40) is brought into contact with the target tissue (64). When a pulse button on the pump is pressed, delivery balloon (36) may be deflated and inflated in a cyclical fashion, based either on parameters that were entered by the user, or on default parameters selected by the pump, which are based on the characteristics of the particular balloon and diameter and/or density measurements made by the system. In this way, the pulse mode of the pump causes the balloon (36) to pulsate according to a desired frequency or change in volume within the balloon, producing a periodically recurring increase and decrease in balloon size. Pulsating is useful for bringing the patch of absorbent (40), charged with a diagnostic and/or therapeutic agent, into cyclical contact with the target tissue (64) within the cavity or to stimulate absorption by the target tissue (64). Even though the absorbent may be in contact with the tissue, the delivery balloon (36) may be inflated further to squeeze the absorbent between the balloon (36) and the wall of the bodily cavity to urge the agent into the tissue.

To further aid in absorption, portions of the delivery balloon (36) not covered with the absorbent (40) may be abrasive. Accordingly, the optional abrading surface of the delivery balloon (36) repeatedly comes into contact with the target tissue (64) to create micro-impacts thereon. As the balloon is deflated and inflated, the abrading members may promote compressive force exhaustion and abrasion to elicit decomposition of tissue or to facilitate infusion or absorption into target tissues (64). Any loose tissue or excess agent may be removed through one or more openings along the catheter (22) located within the chamber (70).

It should be understood that the foregoing is illustrative and not limiting, and that obvious modifications may be made by those skilled in the art without departing from the spirit of the invention. Accordingly, reference should be made primarily to the accompanying claims, rather than the foregoing specification, to determine the scope of the invention.

## Claims

1. A balloon catheter for supplying a therapeutic and/or diagnostic agent to tissue, comprising:
a catheter having a distal end and a proximal end;
a delivery balloon positioned near the distal end of said catheter, said delivery balloon having an expandable material disposed thereon;
said expandable material being at least partially charged with a therapeutic and/or diagnostic agent;
a proximal balloon positioned proximal of said delivery balloon and a distal balloon positioned distal of said delivery balloon;
a first lumen in said catheter through which fluid is supplied to said delivery balloon for inflating said delivery balloon until said expandable material adopts an expanded state against the tissue within a bodily cavity; and
at least one additional lumen through which fluid is supplied to said proximal and distal balloons for inflating said proximal and distal balloons;
said proximal and distal balloons, when inflated, creating a chamber between the proximal balloon and the distal balloon.

2. The balloon catheter of claim 1, wherein said expandable material is a stent.

3. The balloon catheter of claim 2, wherein said expandable material further comprises an absorbent disposed on at least a portion of said stent.

4. The balloon catheter of claim 1, wherein said expandable material comprises an absorbent.

5. The balloon catheter of claim 4, wherein said absorbent adopts a rigid shape within a bodily cavity when cured.

6. The balloon catheter of claim 5, further comprising a probe for applying a curing treatment to said absorbent.

7. The balloon catheter of claim 4, wherein said absorbent is retained on said delivery balloon in a contracted state with at least one string, said at least one string being adapted to break when said delivery balloon is inflated.

8. The balloon catheter of claim 1, wherein said expandable material further comprises a micro RF tag.

9. The balloon catheter of claim 1, further comprising at least one fluid source that supplies fluid to said delivery, proximal and distal balloons.

10. The balloon catheter of claim 9, wherein said at least one fluid source further comprises at least one vacuum source that evacuates fluid from said delivery, proximal and distal balloons.

11. A balloon catheter for supplying a therapeutic and/or diagnostic agent to tissue, comprising:
a catheter having a distal end and a proximal end;
a delivery balloon positioned near the distal end of said catheter, said delivery balloon having an expandable material disposed on at least a portion thereof;
said expandable material comprising said absorbent being at least partially charged with a therapeutic and/or diagnostic agent; and
a first lumen in said catheter through which fluid is supplied to said delivery balloon for inflating said delivery balloon until said expandable material adopts an expanded state against the tissue within a bodily cavity.

12. The balloon catheter of claim 11, wherein said absorbent comprises a sleeve around said delivery balloon.

13. The balloon catheter of claim 11, wherein said absorbent adopts a rigid shape within a bodily cavity when cured.

14. The balloon catheter of claim 13, further comprising a probe for applying a curing treatment to said expandable material.

15. The balloon catheter of claim 11, wherein said absorbent is retained on said delivery balloon in a contracted state with at least one string, said at least one string being adapted to break when said delivery balloon is inflated.
